(19) Europäisches Patentamt
European Patent Office
Office européen des brevets

(11) **EP 3 372 692 A1**

(12) **EUROPEAN PATENT APPLICATION**

(43) Date of publication:
**12.09.2018 Bulletin 2018/37**

(51) Int Cl.:
***C12Q 1/68*** (2018.01)     ***G01N 33/574*** (2006.01)

(21) Application number: **17160303.8**

(22) Date of filing: **10.03.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Friedrich-Alexander-Universität Erlangen-Nürnberg
91054 Erlangen (DE)**

(72) Inventors:
• **Baur, Andreas
91054 Erlangen (DE)**
• **Ostalecki, Christian
92318 Neumarkt i.d.OPf. (DE)**
• **Schuler, Gerold
91080 Spardorf (DE)**

(74) Representative: **Wichmann, Hendrik
Wuesthoff & Wuesthoff
Patentanwälte PartG mbB
Schweigerstraße 2
81541 München (DE)**

(54) **A METHOD FOR CHARACTERIZING MELANOCYTIC LESIONS**

(57)     The present disclosure pertains to the identification of novel biomarkers for the characterization of melanocytic lesions as can be found in skin diseases such as melanomas and psoriasis. In particular, the present invention is directed to a method for characterizing melanocytic lesions and their use in an automated or semi-automated device for the diagnosis and/or monitoring of benign, pre-malignant, and malignant melanocytic lesions, as further defined in the claims.

**EP 3 372 692 A1**

**Description**

The present disclosure pertains to the identification of novel biomarkers for the characterization of melanocytic lesions as can be found in skin diseases such as melanomas and psoriasis. In particular, the present invention is directed to a method for characterizing melanocytic lesions and their use in an automated or semi-automated device for the diagnosis and/or monitoring of benign, pre-malignant, and malignant melanocytic lesions, as further defined in the claims.

**BACKGROUND OF THE INVENTION**

Human tissue is the most important source of biomarkers in health and disease, yet its analysis relies largely on classical histology which in essence is a hundred years old. At present histological diagnosis is still the gold standard in tissue analysis (pathology) supporting disease management in a crucial function, confirming or correcting the diagnosis when clinical appearance is inconclusive. In particular for tumour diagnosis and tumour classification (malignant/benign), histology is indispensable in everyday clinical practice. In addition, it clues to select the proper therapy.

Contrary to its importance, classical histology is quite superficial in its description (e.g. "diffuse proliferation" or "pleomorphic"), and highly dependent on the experience of the histologist. In addition, it is often unable to discriminate between subtypes/variants of a disease, a requirement for personalized medicine. In view of growing clinical needs, immunohistochemistry has gradually entered histological diagnosis. However, until today it remains merely in a supportive role usually restricted to stains with one antibody because of time consuming procedures. In basic science (e.g. animal model research) a maximum of 3-4 antibodies are applied for similar reasons.

Meanwhile multi-antigen tissue staining technologies have been developed and enter the market, able to stain up to 10 antigens on a single tissue section. These technologies, also coined as digital pathology, are likely to change and advance clinical pathology. In the future the presence or absence of tissue biomarkers/antigens will likely influence histopathological diagnosis in particular in cases when clinical procedures and therapies depend on it.

In malignant melanoma tissue antigens like HMB45 or S100 are assessed by single-antigen immunofluorescence procedures and are used to diagnose the cancer. However, these markers cannot discriminate between malignant and benign lesions. So far no histological marker exists that would reliably discriminate benign from malignant melanocytes. Frequently many sections of the lesion in question have to be analyzed sequentially in order to come to a definite diagnostic result. Today the discrimination of early malignant melanoma from pre-malignant lesions is thus time-consuming and requires great expert experience.

Accordingly, there is a need in the art for new methods which allow an easy characterization of melanocytic lesions, such that it can be automatized and is independent from the experience of the histologist. Moreover, there is a need in the art for new biomarkers or a signature of biomarkers which allows characterizing a melanocytic lesion as malignant, pre-malignant or benign.

**SUMMARY OF THE INVENTION**

In order to identify critical transformation events in tissue characterizing the transition from a pre-malignant to a fully malignant melanocytic lesion, the inventors systematically analyzed human skin section from benign nevi to invasive melanoma using a robot-automated staining technology that allows the staining of one tissue section with up to 100 antibodies and more. Using this technology, called multi-epitope ligand cartography (MELC), the inventors could not detect a combination of markers that would reliably discriminate a malignant *melanoma cell* from a premalignant *melanocyte.* However, the inventors discovered that the surrounding *keratinocyte* changed their protein expression pattern (PEP) reflecting the transformation process step-by-step. This was possible because melanocytes are in direct contact with keratinocytes and constantly transfer protein cargo including activated effectors like proteases and kinases. During the transformation process this protein cargo changes considerably, leading to a modulation of the PEP in the recipient cell. Thus we could determine a PEP in keratinocytes characteristic for premalignant nevi that was different from a PEP of keratinocytes surrounding a malignant melanoma cell. The inventors thus identified 7-10 markers to discriminate benign from pre-malignant and malignant melanocytic lesions.

As keratinocytes by far outnumber melanocytes (~30:1) they offer a topographical large tissue area that is easy to scan by naked eye or software programs, particularly when diseased (e.g. malignant) melanocytes are low in number. The presence as well as the absence of these markers and combinations thereof will give a reliable diagnosis even when given by less experienced experts. Furthermore, it will reduce the often time-consuming procedures and examination of multiple tissue sections. The great benefit of this invention is exemplified in malignant melanoma where it is able to discriminate pre-malignant lesions, as for example dysplastic nevi, from truly malignant melanoma cells. However, a similar result was obtained when the inventors analyzed psoriatic lesions. Again the inventors identified a small combination of markers in keratinocytes that was very typical for a psoriatic lesion and absent in healthy skin. Like in Melanoma, a closer analysis suggested that the associated melanocytes had changed their protein cargo leading to an alteration of the keratinocyte PEP. The inventors therefore suggest that any skin condition that typically involve and affect skin melanocytes, e.g. by affecting the homeostasis of melanocytes, will lead to an alteration of the keratinocyte PEP, yielding a disease-specific marker profile.

More specifically, the present invention relates to a method for characterizing melanocytic lesions, comprising determining the expression level of one or more biomarkers of keratinocytes surrounding melanocytes in a biopsy sample obtained from a subject, wherein the one or more biomarker is selected from the group consisting of ADAM10, Notch1, p27[KIP1], CD63, PPARγ, TAP73, and SPPL3; wherein AD-AM10, Notch1, and p27[KIP1] is present in healthy keratinocytes and also when a premalignant melanocytic lesion develops, but disappears when a malignant melanocytic lesion develops; and wherein CD63, PPARγ, TAP73, and SPPL3 is not present in healthy keratinocytes and appears when a pre-malignant or malignant melanocytic lesion develops, wherein the expression level of said biomarker is stronger when a malignant melanocytic lesion develops as compared to when a pre-malignant melanocytic lesion develops; thereby characterizing the melanocytic lesion as a pre-malignant and/or malignant melanocytic lesion; as further defined in the claims. With this invention, including a list of specific tissue markers (n=7-10), this time-consuming diagnostic procedure is standardized, easy to diagnose by less experienced experts and suitable for automated staining and scanning platforms in the new age of digital pathology. The present invention allows the early and precise diagnosis of acute and malignant skin diseases that involve melanocytes, different clinical stages and diseases predisposing conditions by naked eye or software-supported and robot-automated staining platforms. It allows simplifying a time consuming and hence expensive procedure and allows less experienced specialists and medical doctors to make an accurate biomarker-based diagnosis, in particular early melanoma. As a consequence, this invention allows the monitoring of a given skin disease and condition, which may reflect therapeutic success or failure, and may allow a biomarker-based prognosis as well as therapeutic prediction.

Accordingly, the present invention further relates to a use of the method disclosed herein in an automated or semi-automated device for the diagnosis and/or monitoring of benign, pre-malignant, and malignant melanocytic lesions, as further defined in the claims.

## DETAILED DESCRIPTION OF THE PREFERRED EMBODIMENTS

The inventors have discovered that melanocyte-associated keratinocytes change their PEP in a characteristic and specific manner, if these melanocytes are affected by disease conditions or by the appearance of transforming mutations. The underlying mechanism is that melanocytes are connected to keratinocytes and constantly transfer highly active effectors. The composition of these effectors changes under the above-mentioned conditions, leading to a modulation of the PEP in the keratinocyte target cell. In the case of melanoma, the keratinocyte PEP is highly characteristic for premalignant as well as truly malignant melanocytes and hence allows the discrimination of respective the skin lesions. In addition, keratinocytes provide a topographically large staining area as one melanocyte is connected to about 30 keratinocytes and thus the diagnostic field becomes considerably larger. In contrast thereto, melanoma cells do not display a PEP that allows discrimination from premalignant nevi. Tissue sections used for histopathological diagnosis contain thousands of topographically organized cells, each of which expresses thousands of proteins (antigens) that can be identified by reaction with a specific antibody. This procedure is called immunostaining, and is generally well-known to the person skilled in the field. Theoretically, a combination of identified antigens characterizes a given cell type and allows a discrimination from another cell types. While this is true for many examples, a reliable marker pattern has not been identified to discriminate melanocytes and melanoma cells.

Looking for critical transformation events that characterize the transition from premalignant nevi to fully malignant melanoma cells, the inventors could confirm this observation. However, the inventors found that the associated keratinocytes change their PEP in a manner that does allow the discrimination of these two cell conditions. This discrimination of benign, premalignant and malignant melanocytic skin lesions is a frequent constellation in skin histopathology and hence of great clinical and forensic importance. This invention describes a tissue antigen pattern consisting of 7-10 markers that are expressed/not-expressed in melanoma/melanocytes-associated keratinocytes, allowing discriminating premalignant from truly malignant melanocytic lesions.

Therefore, the present invention is directed to a method for characterizing melanocytic lesions, comprising determining the expression level of one or more biomarkers of keratinocytes surrounding melanocytes in a biopsy sample obtained from a subject, wherein the one or more biomarker is selected from the group consisting of ADAM10, Notch1, p27[KIP1], CD63, PPARγ, TAP73, and SPPL3;

wherein ADAM10, Notch1, and p27[KIP1] is present in healthy keratinocytes and when a pre-malignant melanocytic lesion develops, but disappears when a malignant melanocytic lesion develops; and

wherein CD63, PPARγ, TAP73, and SPPL3 is not present in healthy keratinocytes and appears when a pre-malignant or malignant melanocytic lesion develops, wherein the expression level of said biomarker is stronger when a malignant melanocytic lesion develops as compared to when a pre-malignant melanocytic lesion develops;

thereby characterizing the melanocytic lesion as a pre-malignant and/or malignant melanocytic lesion.

As noted above, the identified biomarkers ADAM10, Notch1, and p27$^{KIP1}$ cannot distinguish between healthy melanocytes and pre-malignant melanocytic lesions, but their disappearance is indicative for the development of a malignant melanocytic lesion. Their expression pattern is thus contrary to the identified biomarkers CD63, PPARγ, TAP73, and SPPL3, which are not present in healthy keratinocytes and which appear when a pre-malignant or malignant melanocytic lesion develops. Therefore, it is preferred that one or more biomarkers of each 'group' is determined. In other words in a preferred embodiment, the expression level of one or more biomarker selected from the group consisting of ADAM10, Notch1, and p27$^{KIP1}$ is determined, and the expression level of one or more biomarker selected from the group consisting of CD63, PPARγ, TAP73, and SPPL3 is determined.

In particular, it is preferred that more than one biomarker of a 'group' is determined. For example, it is preferred that the expression level of at least two biomarkers (such as two) selected from the group consisting of ADAM10, Notch1, and p27$^{KIP1}$ is determined, more preferably wherein the expression level of all three biomarkers AD-AM10, Notch1, and p27$^{KIP1}$ is determined. Likewise, it is preferred that the expression level of at least two biomarkers (such as two biomarkers) selected from the group consisting of CD63, PPARγ, TAP73, and SPPL3 is determined; preferably the expression level of at least three biomarkers (such as of three biomarkers) selected from the group consisting of CD63, PPARγ, TAP73, and SPPL3 is determined; and more preferably the expression level of all four biomarkers selected from the group consisting of CD63, PPARγ, TAP73, and SPPL3 is determined.

It is preferred that the expression level of at least two (such as two) of said biomarkers selected from the group consisting of ADAM10, Notch1, p27$^{KIP1}$, CD63, PPARγ, TAP73, and SPPL3 is determined (being from the same 'group' or not), preferably the expression level of at least three (such as three) of said biomarkers, more preferably the expression level of at least four (such as four) of said biomarkers, more preferably the expression level of at least five (such as five) of said biomarkers, still more preferably the expression level of at least six (such as six) of said biomarkers, and most preferably the expression level of all seven biomarkers is determined.

The reliability of the method can be further increased by determining the expression level of additional biomarkers. Thus, in a particular embodiment, further the expression level of APBB1, CD71, or both APPB1 and CD71 is determined, wherein APPB1 and CD71 is not present in healthy keratinocytes and appears when a pre-malignant or malignant melanocytic lesion develops, wherein the expression level of said biomarker is stronger when a malignant melanocytic lesion develops as compared to when a pre-malignant melanocytic lesion develops. In addition, or alternatively, the method may further comprise a step wherein further the expression level of CD66abce is determined, wherein CD66abce is not present in healthy keratinocytes and pre-malignant melanocytic lesion, but appears when a malignant melanocytic lesion develops. In addition, or alternatively, the method may further comprise a step wherein further the expression level of IFN-α is determined, wherein IFN-α is not present in healthy keratinocytes, but is present when a benign melanocytic lesion develops.

Methods for determining the expression level of a biomarker in or on a cell in question are generally known in the art. Depending on the method of choice, it may be helpful to isolate the keratinocytes from the biopsy sample, e.g. by using antibodies against keratinocyte cell-markers coupled to magnetic beads or by using a cell sorter. Such methods are generally known in the art. The expression level may be determined on the RNA level, but is preferably determined on the protein level.

On the RNA level, it is first required to isolate RNA from the keratinocytes of the biopsy sample, using routine methods and kits in the art. The RNA may or may not be then reversely transcribed into cDNA using reverse transcriptase, as is also generally known in the art and commercialized by kits.

For example, RNA may be extracted from biopsy samples by mechanical homogenization and purification using RNeasy Fibrous Tissue Mini Kits (Qiagen). RNA quality and integrity verified by agarose gel electrophoresis and staining with SYBR Green II (Fluka), and RNA is quantified spectrophotometrically. Total RNA aliquots may be reverse transcribed using, for example, MuLV Reverse Transcriptase (Applied Biosystems). As a control of the quality of the reverse transcription, a house-keeping gene such as 18S ribosomal RNA shall be included in the further analysis. Any of these steps can be carried out fully or semi-automatized. Detection may then be carried out by, e.g. using labeled probes, such as molecular beacons, which will bind to the mRNA of said biomarkers, the sequence of which for several different species is available in public gene bank databases (see, for example, ADAM10: A0AV88; Notch1: Q6IAD4; p27KIP1: P46527; CD63: P08962; PPARγ: P37231; TAP73: Q6UNX2; SPPL3: Q8TCT6; CD66abce: NP_001806.2; APPB1: P05067; CD71: P02786; IFNalpha: P01562). Alternatively, the mRNA or cDNA may be subjected to quantitative PCR, such as real-time PCR using primers which specifically bind to the mRNA or the corresponding cDNA of said biomarkers. Primers may be designed using the publicly available nucleotide sequences of the biomarkers, and publicly available programs such as Primer 3 (http://simgene.com/Primer3). Designing sequence primers using such programs represents a routine measure for the skilled person. Accordingly, in one embodiment, the expression level of the one or more biomarker is determined by PCR, in particular real-time PCR, on RNA isolated from keratinocytes which have been isolated from said biopsy sample, which RNA has been reversely transcribed prior to PCR.

More preferably, the expression level of said one or more biomarker is determined on the protein level, for example by immunostaining, in particular by multi-epitope-ligand-cartography (MELC) such as by using MELC robot microscopy. The robot-automated multi-epitope-ligand-cartography (MELC) technology, described in more detail in Schubert et al. Nat. Biotechnol. 24, 1270 (2006), the content of which is incorporated herein by reference, permits the side-by-side staining of tissue sections with up to 100 antibodies and more, allowing to assess topographical PEPs in tissue sections. For example, the following antibodies have been found suitable for determining the expression level: ADAM10 (163003); Notch1 (mN1A); p27[KIP1] (11F10); CD63 (MEM-259); PPARγ (E-8); TAp73 (5G7); SPPL3 (6A2); CD66abce (TET2); APPB1 (1G6); CD71 (M-A712); IFNα (LT27:295). Said antibodies are available from commercial suppliers such as Thermo Fisher Scientific, abcam, Santa Cruz Biotechnology, Miltenyi Biotec, BD Biosciences, and Millipore. However, one may also use other antibodies directed against the above-mentioned proteins, preferably monoclenal antibodies, provided they are suitable for immunostaining applications. Using immunostaining for determining the expression level has the further advantage, that intracellular localization of SSPL3 is further indicative for characterizing the melanocytic lesion as being pre-malignant or malignant: the expression of SSPL3 is primarily perinuclear when a malignant melanocytic lesion develops, and primarily cytosolic when a pre-malignant melanocytic lesion develops.

The histopathological analysis of skin tissue samples from humans or animals follows procedures that have been established for decades. After biopsy was taken, the tissue sample is placed in 4 % paraformaldehyde and embedded in paraffin (FFPE tissue). For additional immunohistochemical analysis (immunostaining), that is usually performed for 1 - 4 antigens, additional sections are cut from the tissue sample. Each of this section is subsequently deparaffinized using xylene and ethanol and subsequently antigen retreval by using EDTA (pH ~ 8 - 9) or citrate buffer (pH ~ 6,0). Preferably for optimal multimarket analysis, for example by the MELC technology, fresh biopsies should be embedded as follows. The excised tissue is embedded completely in O.C.T. Tissue Tek and snap frozen in isopropyl alcohol at -80 °C for 5 - 10 min.

After a tissue section has been cut, a sample by the size of 5 μm is placed on a glass slide. The fixation of the tissue sample is done in acetone at -20 °C for 10 min. After air drying the sample is rehydrated in PBS for 5 min. For control a healthy tissue section can be placed next to the diseased (melanoma) sample.

Subsequently the staining procedure is automatically performed by a cycling process including fluorescence tagging, washing, imaging and photo bleaching using 7 to 10 antibodies. The obtained digital data set is then processed by pixel alignment and flat-field correction of background and illumination faults.

Alternatively, individual tissue sections may be stained by hand for each of the above listed antibodies.

Alternatively, if O.C.T.-embedded/fresh cryofrozen tissue is not available, FFPE (formaline fixed paraffine embedded) tissue sections could be used as described above. The commercial availability of antibodies recognizing antigens in this type of fixated tissue is reduced. However the staining procedure for example by the MELC technology or other procedures is not compromised. Likewise, and as discussed above, individual tissue sections could be stained for each above listed antibodies separately. Preferably, the results of the multi-marker analysis are obtained by MELC technology software, because of its ability to overlay digital images and obtain co-localizations of individual markers in individual tissue cells. Alternatively any multi-marker analysis platform and analysis software could be employed as well as the naked eye analyzing individual digital images side by side.

MELC technology-obtained phase-contrast images and fluorescence images produced after each antibody stain are aligned pixel wise and are corrected for illumination faults using flat-field correction. The alignment should reach a resolution of ±1 pixel. Post bleaching images are subtracted from the following fluorescence tag images. Superimposed images composed a n epitope expression in relation to each pixel (900 × 900-nm2 area) of a visual field (1024 × 1024 pixels).

After the MELC staining procedure, the relative expression level of an antigen can be determined in several tissue areas by assessing the grey value intensity relative to the background. Values can be obtained using the following equation:

$$RFI(AG)_X = \frac{\sum_{i=1}^{n} MGV(AG)_i}{n} - \frac{\sum_{j=1}^{m} IntDen(BG)_j}{\sum_{j=1}^{m} A_j}$$

RFI: Relative Fluorescence Intensity. AG: Antigen. MGV: Mean grey value. IntDent: Integrated density. BG: Background. ROI: Regions of interest.

The assessment of tissue biomarkers is far superior to the conventional and highly descriptive histopathological analysis of skin tissue sections, which relies entirely on the expertise of the pathologist and is not supported by objective criteria. Hence the development of marker profiles for the diagnosis of skin diseases, as well as other diseases, is not only necessary but a predictable development.

The respective expression levels are compared to expression levels found in healthy keratinocytes. This may be done by including a comparative sample, or by comparing the outcome to values previously obtained from healthy keratinocytes. The healthy keratinocytes may origin from the subject from which the biopsy sample has been taken, or from one or more other healthy individuals of the same species. If the method is implemented for automatization, expression levels of healthy keratinocytes may be provided by way of a database or a software program, such that the whole analysis of the expression levels can be automatized.

Preferably the biopsy sample is a skin tissue biopsy sample of the subject. However, other tissue biopsy samples are also contemplated as long as they contain a melanocytic lesion comprising keratinocytes. For example, said biopsy sample may be a lymph node biopsy sample.

The subject is usually a mammal. While its use in the veterinary field is not excluded, it is preferred that the mammal is a human. The method is of particular interest for those subjects, who suffer or are suspected to suffer from a skin disease involving melanocytes. Such diseases can be easily identified by abnormal growth of melanocytes, as for example in malignant melanoma or psoriasis. Preferably the subject has or is suspected to have a malignant melanoma.

The new procedure described here represents a significant advancement for the detection, diagnosis and monitoring of malignant melanoma and is both suitable and necessary for semi-automated procedures and diagnostic read-outs in digital pathology. Accordingly, in a preferred embodiment, the evaluation of the determined expression levels is automated by a software-supported manner. The presently disclosed method is particularly useful for implementation in an automated or semi-automated device for the diagnosis and/or monitoring of benign, pre-malignant, and malignant melanocytic lesions.

Further information with regard to the biomarkers is provided in the following Table 1. The table summarizes the topographical and subcellular changes of the keratinocyte PEP from healthy epidermis to melanoma-associated epidermis as assessed by MELC analyzing the indicated factors. The results are representative for the analysis of 6 SSM and 6 samples of healthy skin.

BL = basal layer; CM = cell membrane; CY = cytoplasm; GL = granular layer; NE = nuclear envelope; NU = nucleus; PN = perinuclear; SL = spinous layer; x = weak signal; **X** = strong signal; [a]: complete loss of staining; [b]: appearance of new factors/staining; [c]: staining in additional subcellular localizations.

| | Healthy skin | | | | | | | | Melanoma | | | | | | | |
| | Localization | | | | | | | | Localization | | | | | | | |
| | subcellular | | | | | layer of epidermis | | | subcellular | | | | | layer of epidermis | | |
| | CM | CY | PN | NE | NU | BL | SL | GL | CM | CY | PN | NE | NU | BL | SL | GL |
|---|---|---|---|---|---|---|---|---|---|---|---|---|---|---|---|---|
| ADAM10 | X | | | | | x[a] | X[a] | X[e] | | | | | | | | |
| APBB1 | | | | | | | | | X | x | | | | x[b] | X[b] | X[b] |
| β-catenin | X | | | | | x | X | x | | X | | | | x | X | X |
| CD63 | | | | | | | | | X | x | | | | x[b] | X[b] | |
| CD66abce | | | | | | | | | X | | | | | | x[b] | X[b] |
| CD71 | | | | | | | | | X | x | | | | X[b] | X[b] | |
| C/EBPβ | | | X | | | | X | X | X | | X | | | | X | X |
| CK2A2 | | x | | | X | x | X | x | X | | | X | | x | X | X |
| p-EBF3 | | X | | | | X | | | X | | | | | X | X[c] | |
| IFN-α | | | | | x | X | X | x | X | | | X | | X | X | x |
| Lamp1 | X | | | | | X | X | X | | x | X | | | x | X | x |
| Nestin | | X | | | | X | | | X | | | | | X | X[c] | |
| Notch1 | | X | | | | x[a] | X[a] | X[a] | | | | | | | | |
| p27KIP1 | | X | | | | x[a] | X[a] | X[a] | | | | | | | | |
| PPARγ | | | | | | | | | X | | | | | x[b] | X[b] | X[b] |
| RIMS3 | | | X | | | X | X | | X | | X | | | X | X | |
| SPPL3 | | | | | | | | | | | | | X | X[b] | X[b] | |
| SYT10 | | X | | | | X | | | X | | | | | X | X[c] | x[c] |
| TAp73 | | | | | | | | | X | | | | | x[b] | X[b] | X[b] |

The invention is further described by way of the following embodiments:

1. A method for characterizing melanocytic lesions, comprising determining the expression level of one or more biomarkers of keratinocytes surrounding melanocytes in a biopsy sample obtained from a subject,
wherein the one or more biomarker is selected from the group consisting of ADAM10, Notch1, p27KIP1, CD63, PPARγ, TAP73, and SPPL3;
wherein ADAM10, Notch1, and p27KIP1 is present in healthy keratinocytes and when a pre-malignant melanocytic lesion develops, but disappears when a malignant melanocytic lesion develops; and

wherein CD63, PPARγ, TAP73, and SPPL3 is not present in healthy keratinocytes and appears when a pre-malignant or malignant melanocytic lesion develops, wherein the expression level of said biomarker is stronger when a malignant melanocytic lesion develops as compared to when a pre-malignant melanocytic lesion develops;

thereby characterizing the melanocytic lesion as a pre-malignant and/or malignant melanocytic lesion.

2. The method of embodiment 1, wherein the expression level of one or more biomarker selected from the group consisting of ADAM10, Notch1, and p27$^{KIP1}$ is determined, and the expression level of one or more biomarker selected from the group consisting of CD63, PPARγ, TAP73, and SPPL3 is determined.

3. The method of embodiment 1 or 2, wherein the expression level of at least two biomarkers selected from the group consisting of ADAM10, Notch1, and p27$^{KIP1}$ is determined, preferably wherein the expression level of all biomarkers selected from the group consisting of ADAM10, Notch1, and p27$^{KIP1}$ is determined.

4. The method of any one of embodiments 1-3, wherein the expression level of at least two biomarkers selected from the group consisting of CD63, PPARγ, TAP73, and SPPL3 is determined; preferably the expression level of at least three biomarkers selected from the group consisting of CD63, PPARγ, TAP73, and SPPL3 is determined; and more preferably the expression level of all biomarkers selected from the group consisting of CD63, PPARγ, TAP73, and SPPL3 is determined.

5. The method of any one of embodiments 1-4, wherein the expression level of at least two of said biomarkers, preferably the expression level of at least three of said biomarkers, more preferably the expression level of at least four of said biomarkers, more preferably the expression level of at least five of said biomarkers, still more preferably the expression level of at least six of said biomarkers, and most preferably the expression level of all seven biomarkers is determined.

6. The method of any one of embodiments 1-5, wherein further the expression level of APBB1, CD71, or both APPB1 and CD71 is determined, wherein APPB1 and CD71 is not present in healthy keratinocytes and appears when a pre-malignant or malignant melanocytic lesion develops, wherein the expression level of said biomarker is stronger when a malignant melanocytic lesion develops as compared to when a pre-malignant melanocytic lesion develops.

7. The method of any one of embodiments 1-6, wherein further the expression level of CD66abce is determined, wherein CD66abce is not present in healthy keratinocytes and pre-malignant melanocytic lesion, but appears when a malignant melanocytic lesion develops.

8. The method of any one of embodiments 1-7, wherein further the expression level of IFN-α is determined, wherein IFN-α is not present in healthy keratinocytes, but is present when a benign melanocytic lesion develops.

9. The method of any one of embodiments 1-8, wherein the expression level of the one or more biomarker is determined by immunostaining, in particular by multi-epitope-ligand-cartography (MELC); or

wherein the expression level of the one or more biomarker is determined by PCR, in particular real-time PCR, on RNA isolated from keratinocytes which have been isolated from said biopsy sample, which RNA has been reversely transcribed prior to PCR.

10. The method of embodiment 9, wherein the expression of SSPL3 is primarily perinuclear when a malignant melanocytic lesion develops and primarily cytosolic when a pre-malignant melanocytic lesion develops.

11. The method of any of embodiments 1-10, wherein said biopsy sample is a skin tissue biopsy sample, or wherein said biopsy sample is a lymph node biopsy sample; preferably wherein said biopsy sample is a skin tissue biopsy sample.

12. The method of any of embodiments 1-11, wherein the subject is a mammal, preferably a human.

13. The method of any of embodiments 1-12, wherein the subject has or is suspected to have a skin disease involving melanocytes, in particular wherein the skin disease involving melanocytes is selected from malignant melanoma or psoriasis, preferably wherein the skin disease involving melanocytes is malignant melanoma.

14. The method of any of embodiments 1-13, wherein the evaluation of the determined expression levels is automated by a software-supported manner.

15. Use of the method according to any one of embodiments 1-14 in an automated or semi-automated device for the diagnosis and/or monitoring of benign, pre-malignant, and malignant melanocytic lesions.

The inventions is further described by reference to the following figures and examples, which are however not to be understood to limit the invention as defined in the claims.

## DESCRIPTION OF THE FIGURES

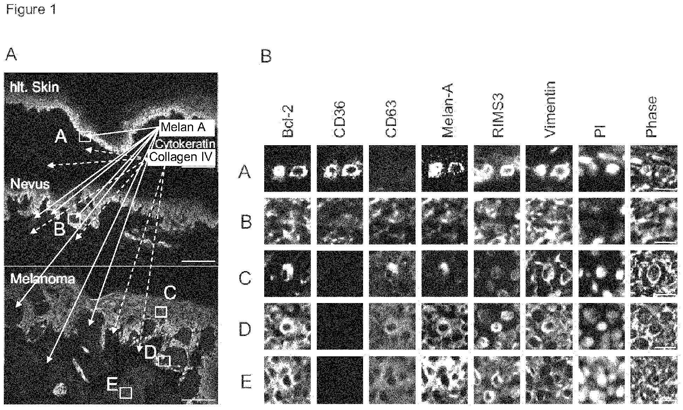

**Figure 1: Malignant, premalignat and benign melanocytic skin lesions have a similar PEP that does not allow a discrimination of these cells.**

Representative analysis of 6 markers by MELC (B) in healthy melanocytes (hlt. skin), a compound nevus and a SSM (stained by Melan-A) shown in (A). Tissue areas depicted in (A) by squares and capital letters were analyzed in (B). The scale bar represents 100 $\mu$m (A) and 10 $\mu$m (B). **Note:** all panels in one horizontal row in (B) depict the same tissue section.

**Figure 2: Melanoma- but not nevi-associated keratinocytes have an altered PEP.**

A compound nevus and an early SSM were stained for the indicated factors by MELC. The antibody directed against APBB1 recognizes the amyloid precursor protein. The scale bar represents 100 $\mu$m.

**Figure 3: Keratinocytes in association with melanoma cells change their PEP**

**(A)** A tissue section representing the transition from healthy epidermis (p27$^{KIP1}$, green) to melanoma (CD63, white) was stained for the indicated markers. Individual cells show an all or nothing staining phenotype for specific markers (right panel; see also Table 1). The scale bar represents 50 $\mu$m. **(B)** Co-culture of melanoma cells (Mel.) with Keratinocytes (Ker.), showing how melanoma cells establish contact with keratinocytes. The scale bar represents 50 $\mu$m.

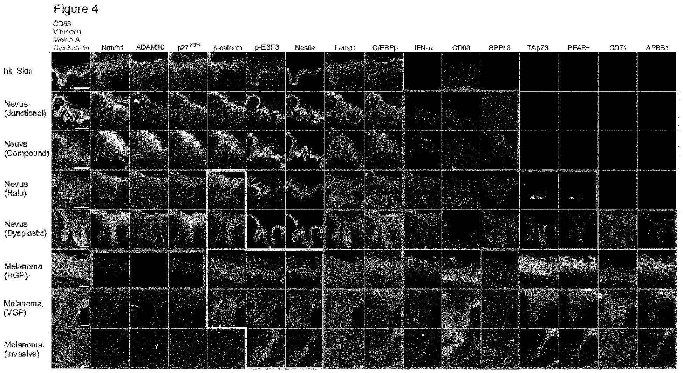

**Figure 4: The keratinocyte PEP reflects the transformation process from benign nevi to invasive melanoma.**

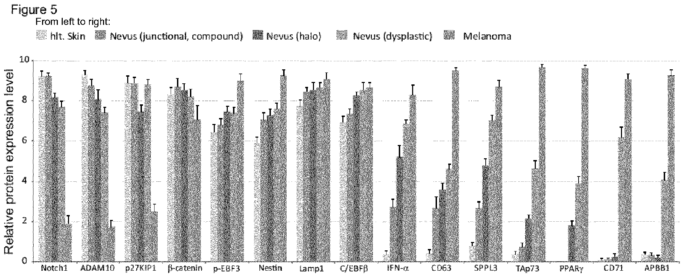

Keratinocyte tissue areas from healthy skin, different nevi and melanomas (left panels) were systematically analyzed for keratinocyte-specific markers using MELC. The most prominent as well as representative results are shown and otherwise summarized in **Figure 5.** Markers either increased in expression (CD63, SPPL3, TAP73, PPAR$\gamma$, CD71, APBB1, IFN$\alpha$), lost expression entirely (Notch1, ADAM10, p27$^{KIP1}$), extended their expression into different epidermal layers ($\beta$-catenin, p-EBF3, Nestin), or changed their subcellular localization (SPPL3, LAMp1, C/EBP$\beta$). The scale bar represents 50 $\mu$m.

**Figure 5: Quantification of the keratinocyte PEP in healthy skin, different nevi and SSM as demonstrated in Figure 4.**

Relative expression levels of proteins were determined in distinct melanocytic lesions (healthy skin, nevi, SSM) by assessing the grey value intensity. Expression levels for a given antigen were determined by calculating the mean from 6 different tissues per lesion. The highest value of these 6 expression levels were set to 10. Error bars represent standard deviations of the mean.

## EXAMPLE

Using a systemic approach with the multi-epitope-ligand-cartography (MELC)-technology, we analyzed protein expression profiles (PEP) in nevi and BRAFV600E+ superficial spreading melanomas (SSM) for key transformation events.

To obtain antibodies applicable in the MELC-technology, 814 randomly selected hybridoma supernatants from the antibody production facility of the Helmholtz-Centre in Munich, and 173 commercially available antibodies were subjected to a screening algorithm to obtain those antibodies giving a specific staining in tissue (epidermis and dermis) for melanoma cells (n=57). We reasoned that key factors of the transformation process would appear in melanomas but not in nevi. We also selected antibodies that were specific for melanoma-associated keratinocytes (n=7) or for melanomas and keratinocytes (n=12). Single tissue sections of 6 BRAFV600E+ SSM, 6 junctional and compound nevi (3/3), and 6 samples of healthy skin were stained by the whole antibody set. For each antigen the average relative expression level was determined using an equation integrating grey value intensities of the background and 6 to 30 different staining areas for each sample.

Surprisingly, almost all antibodies gave a positive staining with melanocytes, nevi and melanoma tissue. There were only few exceptions, as for example CD63 was detected only in nevi and melanoma, and CD36 was only found in melanocytes and nevi **(Figure 1).** However, the relative expression level of selected antigens differed significantly. Nevi showed a >2-fold increase in 6/57 antigens over melanocytes, and melanoma cells further increased expression levels in 10/57 antigens, including those increased in nevi, like CD63 and CD71. A number of proteins were also reduced in concentration >2-fold in nevi compared to melanocytes (12/57), and in melanoma cells compared to nevi (6/57). Thus protein expression levels of key proteins changed rather uniformly across different samples and potentially had a role in the transformation process.

To substantiate this assumption we compared PEPs in three different layers of the SSMs, namely in the basal (BL), apical (AL) and dermal layer (DL), as DL cells have a more aggressive growth behavior. However, these results did not point to crucial events of the transformation process.

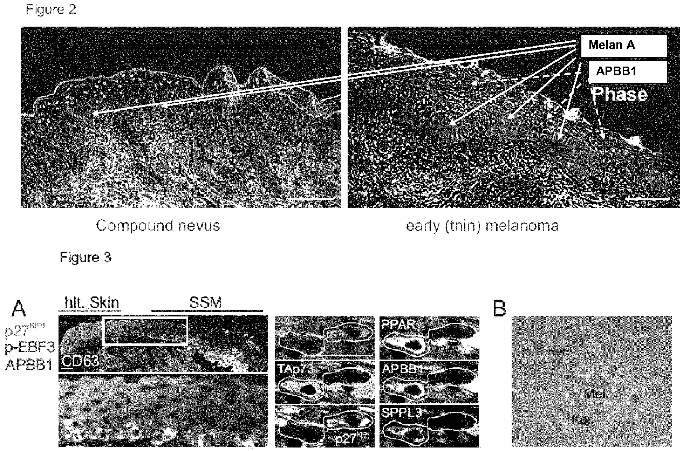

We noticed that the PEP of keratinocytes adjacent to SSM, but not to benign nevi, changed visibly, and followed an all or nothing expression phenotype for numerous factors **(Figure 3).** To confirm this finding we cultured different primary melanoma cells (ML01, -03, -05 ,-07, -11) characterized previously (Lee te al. Mol. Cell 49, 668 (2013)) with keratinocytes (HaCaT cells), and noticed that all melanoma cells formed dendritic connections. We placed melanoma cells on one side of a keratinocyte colony and analyzed their PEP by MELC after 72h. In a gradient type fashion, the keratinocytes changed their PEP and the subcellular localization of selected factors. These changes were very similar to those seen between nevi and SSM in tissue sections. A systematic analysis of melanoma cell- and SSM-associated keratinocytes (n=6 each condition) confirmed their overall similar PEP. Thus the transfer of certain effectors to keratinocytes was a conserved function of melanoma cells.

In view of these results we speculated that melanocyte transformation occurred in discernable steps, potentially mirrored by the keratinocyte PEP. Thus we systematically compared keratinocyte PEPs in healthy skin, junctional-, compound-, halo- and dysplastic nevi, as well as in BRAFV600E+ SSMs with horizontal, vertical and invasive growth patterns. Representative results of each tissue are shown in **Figure 4,** revealing a progressive change of the keratinocyte PEP from healthy skin to melanoma. This change was characterized by factors that seemingly disappeared (Notch1, AD-AM10, p27$^{KIP1}$), gradually appeared and increased their expression (CD63, SPPL3, TAP73, PPARγ, CD71, APBB1, IFNα), or changed their subcellular localization (SPPL3). While changes started at the level of benign nevi with low expression levels of IFN-α, CD63 and SPPL3, major differences were seen between the dysplastic nevi and the early SSM. This included the seemingly complete loss of p27$^{KIP1}$, ADAM10 and its substrate Notch1. Changes in expression levels is also shown in **Figure 5,** and can be summarized as follows:

**1) Malignant lesions**

Markers that are present in healthy keratinocytes and disappear when a malignant lesion develops:

ADAM10 (antibody recognizing the N-terminus of ADAM10), Notch1, p27$^{KIP1}$ Markers that are not present in healthy keratinocytes and appear upon malignant transformation:

APBB1 (Amyloid precursor protein), CD63, CD66abce, CD71, PPAR TAP73, SPPL3 (perinuclear region)

Biomarker summary malignant transformation:

ADAM10 (N-terminus) ↓↓, Notch1 ↓↓, p27$^{KIP1}$ ↓↓, APBB1 ↑↑, CD63 ↑↑, CD66abce ↑↑, CD71 ↑↑, PPAR· ↑↑, TAP73 ↑↑, SPPL3 (perinuclear) ↑↑.

Minimal Biomarker combination in keratinocytes for malignant lesions:

ADAM10 (N-terminus) ↓↓, Notch1 ↓↓, p27$^{KIP1}$ ↓↓, CD63 ↑↑, PPARg ↑↑, TAP73 ↑↑, SPPL3 (perinuclear) ↑↑.

**2) Pre-malignant lesions**

Markers that are present in healthy keratinocytes and disappear in premalignant melanocytic lesions:

None.

Markers that are not present in healthy keratinocytes and appear in premalignant lesions:

APBB1 ↑, CD71↑, PPAR· ↑, TAP73 ↑, SPPL3 (cytoplasmic) ↑↑.

Biomarker summary premalignant melanocytic lesion:

CD66abce ↓↓, CD63 ↓↓, ADAM10 (N-terminus) ↑↑, Notch1 ↑↑, p27$^{KIP1}$ ↑↑, SPPL3 (cytoplasmic) ↑↑, APBB1 ↑, CD71 ↑, PPAR· ↑, TAP73 ↑.

Minimal Biomarker combination in keratinocytes for malignant lesions

CD63 ↓↓, ADAM10 (N-terminus) ↑↑, Notch1 ↑↑, p27$^{KIP1}$ ↑↑, SPPL3 (cytoplasmic) ↑↑, PPAR· ↑, TAP73 ↑.

**3) Benign melanocytic lesion**

Markers that are present in healthy keratinocytes and disappear in benign melanocytic lesions:

None.

Markers that are not present in healthy keratinocytes and appear in benign melanocytic lesions:

IFN-$\alpha$ ↑.

Biomarker summary benign metanocytic lesion:

IFN-$\alpha$ ↑.

Surprisingly we found that more variables were involved in melanocyte transformation than appreciated so far, including protein expression levels, their subcellular localization and potentially the early keratinocyte environment. Keratinocytes with an altered PEP may stimulate transforming melanocytes, similar as they stimulate melanin production after sun exposure. The reciprocal transfer of new effectors may occur through cell-cell contact, cell dendrites as shown here, or alternatively through endosomal secretion or transcytosis. While we have no direct proof for such a reciprocal cell interaction, such a scenario would be consistent with the often slow changes seen in premalignant melanocytic lesions.

The here reported insights into the early melanoma transformation events were obtained through a multi-antigen assessment in tissue. The topographical allocation of PEPs was a key in our approach and a major difference to other multi-antigen approaches as for example mass spectrometry. The protein markers demonstrated in this study in melanoma-associated keratinocytes, could be used for the diagnosis of early melanomas and the discrimination of dysplastic lesions from truly transformed melanocytes.

**Claims**

1. A method for characterizing melanocytic lesions, comprising determining the expression level of one or more biomarkers of keratinocytes surrounding melanocytes in a biopsy sample obtained from a subject,
   wherein the one or more biomarker is selected from the group consisting of ADAM10, Notch1, p27$^{KIP1}$, CD63, PPAR$\gamma$, TAP73, and SPPL3;
   wherein ADAM10, Notch1, and p27$^{KIP1}$ is present in healthy keratinocytes and when a pre-malignant melanocytic lesion develops, but disappears when a malignant melanocytic lesion develops; and
   wherein CD63, PPAR$\gamma$, TAP73, and SPPL3 is not present in healthy keratinocytes and appears when a pre-malignant or malignant melanocytic lesion develops, wherein the expression level of said biomarker is stronger when a malignant melanocytic lesion develops as compared to when a pre-malignant melanocytic lesion develops;
   thereby characterizing the melanocytic lesion as a pre-malignant and/or malignant melanocytic lesion.

2. The method of claim 1, wherein the expression level of one or more biomarker selected from the group consisting of ADAM10, Notch1, and p27$^{KIP1}$ is determined, and the expression level of one or more biomarker selected from the group consisting of CD63, PPAR$\gamma$, TAP73, and SPPL3 is determined.

3. The method of claim 1 or 2, wherein the expression level of at least two biomarkers selected from the group consisting of ADAM10, Notch1, and p27$^{KIP1}$ is determined, preferably wherein the expression level of all biomarkers selected from the group consisting of ADAM10, Notch1, and p27$^{KIP1}$ is determined.

4. The method of any one of claims 1-3, wherein the expression level of at least two biomarkers selected from the group consisting of CD63, PPAR$\gamma$, TAP73, and SPPL3 is determined; preferably the expression level of at least three biomarkers selected from the group consisting of CD63, PPAR$\gamma$, TAP73, and SPPL3 is determined; and more preferably the expression level of all biomarkers selected from the group consisting of CD63, PPAR$\gamma$, TAP73, and SPPL3 is determined.

5. The method of any one of claims 1-4, wherein the expression level of at least two of said biomarkers, preferably the expression level of at least three of said biomarkers, more preferably the expression level of at least four of said biomarkers, more preferably the expression level of at least five of said biomarkers, still more preferably the expression level of at least six of said biomarkers, and most preferably the expression level of all seven biomarkers is determined.

6. The method of any one of claims 1-5, wherein further the expression level of APBB1, CD71, or both APPB1 and CD71 is determined, wherein APPB1 and CD71 is not present in healthy keratinocytes and appears when a pre-malignant or malignant melanocytic lesion develops, wherein the expression level of said biomarker is stronger when a malignant melanocytic lesion develops as compared to when a pre-malignant melanocytic lesion develops.

7. The method of any one of claims 1-6, wherein further the expression level of CD66abce is determined, wherein CD66abce is not present in healthy keratinocytes and pre-malignant melanocytic lesion, but appears when a malignant melanocytic lesion develops.

8. The method of any one of claims 1-7, wherein further the expression level of IFN-$\alpha$ is determined, wherein IFN-$\alpha$ is not present in healthy keratinocytes, but is present when a benign melanocytic lesion develops.

9. The method of any one of claims 1-8, wherein the expression level of the one or more biomarker is determined by immunostaining, in particular by multi-epitope-ligand-cartography (MELC); or
wherein the expression level of the one or more biomarker is determined by PCR, in particular real-time PCR, on RNA isolated from keratinocytes which have been isolated from said biopsy sample, which RNA has been reversely transcribed prior to PCR.

10. The method of claim 9, wherein the expression of SSPL3 is primarily perinuclear when a malignant melanocytic lesion develops and primarily cytosolic when a pre-malignant melanocytic lesion develops.

11. The method of any of claims 1-10, wherein said biopsy sample is a skin tissue biopsy sample, or wherein said biopsy sample is a lymph node biopsy sample; preferably wherein said biopsy sample is a skin tissue biopsy sample.

12. The method of any of claims 1-11, wherein the subject is a mammal, preferably a human.

13. The method of any of claims 1-12, wherein the subject has or is suspected to have a skin disease involving melanocytes, in particular wherein the skin disease involving melanocytes is selected from malignant melanoma or psoriasis, preferably wherein the skin disease involving melanocytes is malignant melanoma.

14. The method of any of claims 1-13, wherein the evaluation of the determined expression levels is automated by a software-supported manner.

15. Use of the method according to any one of claims 1-14 in an automated or semi-automated device for the diagnosis and/or monitoring of benign, premalignant, and malignant melanocytic lesions.

Figure 1

Figure 2

Melan A

APBB1

Phase

Compound nevus                    early (thin) melanoma

Figure 3

A    hlt. Skin        SSM

p27^KIP1
p-EBF3
APBB1    CD63

PPARγ

TAp73    APBB1

SPPL3
p27^KIP1

B

Ker

Mel.

Ker

## Figure 4

Figure 5

From left to right:

hlt. Skin　　Nevus (junctional, compound)　　Nevus (halo)　　Nevus (dysplastic)　　Melanoma

Europäisches
Patentamt

European
Patent Office

Office européen
des brevets

# EUROPEAN SEARCH REPORT

Application Number

EP 17 16 0303

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DANIELA MASSI ET AL: "EVIDENCE FOR DIFFERENTIAL EXPRESSION OF NOTCH RECEPTORS AND THEIR LIGANDS IN MELANOCYTIC NEVI AND CUTANEOUS MALIGNANT MELANOMA", MODERN PATHO, NATURE PUBLISHING GROUP, GB, vol. 19, 1 January 2006 (2006-01-01), pages 246-254, XP008077878, ISSN: 0893-3952, DOI: 10.1038/MODPATHOL.3800526 * page 249, column 2, paragraph 2; figures 1,2 * | 1-15 | INV. C12Q1/68 G01N33/574 |
| A | SOPHIA BOYOUNG LEE ET AL: "PAX2 Regulates ADAM10 Expression and Mediates Anchorage-Independent Cell Growth of Melanoma Cells", PLOS ONE, vol. 6, no. 8, 18 August 2011 (2011-08-18), page e22312, XP055397435, DOI: 10.1371/journal.pone.0022312 * abstract * | 1-15 | |
| A | BAUR A S ET AL: "SPPL3-mediated ADAM10 Activation is a Key Step in the evolution of BRAFV600E+Superficial Spreading Melanomas (SSM)", THE JOURNAL OF INVESTIGATIVE DERMATOLOGY : OFFICIAL JOURNAL OF THE SOCIETY FOR INVESTIGATIVE DERMATOLOGY AND THE EUROPEAN SOCIETY FOR DERMATOLOGICAL RESEARCH, ELSEVIER, US, vol. 136, no. 9, Suppl. 2, 31 August 2016 (2016-08-31), page S247, XP009500073, ISSN: 0022-202X * abstract * | 1-15 | TECHNICAL FIELDS SEARCHED (IPC)  C12Q G01N |

-/--

The present search report has been drawn up for all claims

| Place of search | Date of completion of the search | Examiner |
|---|---|---|
| The Hague | 10 August 2017 | Gundlach, Björn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 17 16 0303

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | OSTALECKI C ET AL: "P252: Chronic activation of keratinocytes by the peptide peptidase like 3 (SPPL3): a crucial factor in melanoma pathogenesis?", EXPERIMENTAL DERMATOLOGY; 41TH ANNUAL MEETING OF THE ARBEITSGEMEINSCHAFT DERMATOLOGISCHE FORSCHUNG (ADF), BLACKWELL MUNSGAARD, COPENHAGEN; DK; COLOGNE, GERMANY, vol. 23, no. 3, 1 March 2014 (2014-03-01), page e43, XP008185545, ISSN: 0906-6705, DOI: 10.1111/EXD.12314 [retrieved on 2014-03-07] * abstract * ----- | 1-15 | |
| T | OSTALECKI CHRISTIAN ET AL: "Multiepitope tissue analysis reveals SPPL3-mediated ADAM10 activation as a key step in the transformation of melanocytes", SCIENCE SIGNALING, AMERICAN ASSOCIATION FOR THE ADVANCEMENT OF SCIENCE, US, vol. 10, no. 470, 14 March 2017 (2017-03-14), pages 1-12, XP009500075, ISSN: 1945-0877, DOI: 10.1126/SCISIGNAL.AAI8288 ----- | | |

TECHNICAL FIELDS
SEARCHED (IPC)

The present search report has been drawn up for all claims

| Place of search | Date of completion of the search | Examiner |
|---|---|---|
| The Hague | 10 August 2017 | Gundlach, Björn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SCHUBERT et al.** *Nat. Biotechnol.,* 2006, vol. 24, 1270
- **LEE.** *Mol. Cell,* 2013, vol. 49, 668